# DEMANDE DE BREVET EUROPEEN

(11) **EP 4 450 051 A1**
(43) Date de publication de la demande: **23.10.2024**
(21) Numéro de dépôt: 23305593.8
(22) Date de dépôt: 18.04.2023
(51) Int. Cl.: A61K 8/365, A61K 8/368, A61K 8/73, A61K 8/34, A61Q 15/00, A61Q 17/00

(54) **COMPOSITION DÉODORANTE À BASE D'ACIDE SALICYLIQUE OU DE DÉRIVÉ D'ACIDE SALICYLIQUE, D'ALPHA HYDROXY ACIDE ET DE CHITOSANE**

(71) Demandeur: L'OREAL, 75008 Paris (FR)
(72) Inventeur: MOINE, Toni, 94550 Chevilly Larue (FR); GOZLAN, Charlotte, 94550 Chevilly Larue (FR)
(74) Mandataire: Lavoix

(57) **Abrégé**

La présente invention concerne une composition, comprenant, dans un milieu aqueux physiologiquement acceptable :
(a) du chitosan natif,
(b) de l'acide salicylique ou l'un de ses dérivés, et
(c) au moins un alpha hydroxy acide,
dans laquelle la teneur en poids en chitosan est strictement supérieure à la teneur en poids en alpha hydroxy acide, et la teneur en poids en alpha hydroxy acide est strictement supérieure à la teneur en poids en acide salicylique ou l'un de ses dérivés, les teneurs en poids étant définies par rapport au poids total de la composition.

Elle concerne également un procédé cosmétique de prévention et/ou de traitement des odeurs coporelles.

## Description

La présente invention concerne une composition cosmétique à base d'acide salicylique ou de dérivé d'acide salicylique, d'alpha hydroxy acide et de chitosan. La présente invention concerne également un procédé cosmétique de prévention et/ou de traitement des odeurs coporelles.

Un certain nombre d'odeurs corporelles humaines sont directement liées à la présence, excessive ou non, de fluides corporels sécrétés au niveau d'un épiderme, en particulier la sueur, et également le sébum. Ainsi, l'évolution des odeurs corporelles est notamment liée à une modification, quantitative et/ou qualitative, des composés et/ou substance présents en surface cutanée, dits encore « substrat cutané », et dont la dégradation par la microflore cutanée résidente est à l'origine de la biosynthèse de composés volatils malodorants. Or, pour des raisons manifestes, les mauvaises odeurs corporelles sont perçues comme socialement invalidantes et, à ce titre, il apparaît important de contrôler efficacement les phénomènes biologiques les générant.

La sueur est un liquide sécrété par deux types de glandes sudoripares, à savoir les glandes eccrines et les glandes apocrines. Qu'elle soit d'origine eccrine ou apocrine, et sous réserve qu'elle soit générée de manière non excessive, la sueur est peu ou pas odorante lors de sa sécrétion. C'est la mise en contact de la sueur, et plus particulièrement de la sueur apocrine avec des bactéries, qui induit la formation de composés volatils et malodorants, via la dégradation bactérienne de cette sueur. La bactérie *Corynebacterium xerosis* est en particulier connue pour être une des principales bactéries à l'origine des mauvaises odeurs corporelles.

A l'image de la sueur, le sébum est également susceptible de faire l'objet d'une dégradation bactérienne, et peut également être générateur de mauvaises odeurs.

Pour lutter contre les mauvaises odeurs, et en particulier celles liées à la transpiration, il a déjà été proposé divers composés qui, par application topique, sont susceptibles d'inhiber la sudation, d'inhiber les bactéries responsables des transformations chimiques décrites ci-dessus, de masquer lesdites odeurs, ou de réduire/détruire la microflore indigène. En particulier, il est connu d'utiliser des compositions comprenant de l'acide salicylique ou un dérivé d'acide salicylique pour détruire notamment la bactérie *Corynebacterium xerosis.*

Cependant, l'efficacité de ces compositions n'est pas totale, et présente en outre une limite dans le temps.

Il existe donc un besoin pour de nouvelles compositions présentant une efficacité améliorée de réduction des odeurs corporelles, en particulier dues à la dégradation bactérienne de la sueur et/ou du sébum.

Il existe également un besoin pour de nouvelles compositions présentant une efficacité de réduction des odeurs corporelles, en particulier dues à la dégradation bactérienne de la sueur et/ou du sébum, qui soit plus durable dans le temps.

Par ailleurs, la formulation de produits cosmétiques respectueux de l'environnement, c'est-à-dire dont la conception et le développement tiennent compte des enjeux environnementaux, devient une préoccupation majeure pour contribuer à relever les défis planétaires.

Il se révèle donc essentiel de proposer des compositions et/ou des procédés de préparation et/ou des ingrédients plus durables permettant ainsi de répondre à ces enjeux environnementaux.

Dans ce contexte, il est important de développer de nouvelles compositions cosmétiques avec une meilleure empreinte carbone notamment en favorisant l'emploi de matières premières renouvelables et/ou avec un bon index de naturalité et/ou d'origine naturelle et plus particulièrement d'origine végétale tout en réduisant l'utilisation de composés d'origine pétrochimique.

La présente invention a donc pour objet une composition, de préférence cosmétique, préférentiellement déodorante, comprenant, dans un milieu aqueux physiologiquement acceptable :
(a) du chitosan natif,
(b) de l'acide salicylique ou l'un de ses dérivés, et
(c) au moins un alpha hydroxy acide,
dans laquelle la teneur en poids en chitosan est strictement supérieure à la teneur en poids, de préférence à la teneur totale en poids, en acide salicylique ou l'un de ses dérivés, et la teneur en poids, de préférence à la teneur totale en poids, en acide salicylique ou l'un de ses dérivés est strictement supérieure à la teneur en poids, de préférence à la teneur totale en poids, en alpha hydroxy acide, les teneurs en poids étant définies par rapport au poids total de la composition.

Les inventeurs ont découvert de manière surprenante que l'ajout de chitosan et d'alpha hydroxy acide (AHA) dans une composition comprenant de l'acide salicylique ou un de ses dérivés exacerbe l'activité antibactérienne de l'acide salicylique et de ses dérivés. En particulier, l'activité antibactérienne de l'acide salicylique et de ses dérivés contre *Corynebacterium xerosis* est exacerbée, et démontre une synergie entre le duo chitosan/AHA et l'acide salicylique et ses dérivés.

Par « physiologiquement acceptable », on entend un milieu compatible avec les matières kératiniques.

La présente invention concerne également l'utilisation cosmétique d'une composition comprenant, dans un milieu aqueux physiologiquement acceptable :
(a) du chitosan natif,
(b) de l'acide salicylique ou l'un de ses dérivés, et
(c) au moins un alpha hydroxy acide,
pour prévenir et/ou réduire les odeurs corporelles.

De préférence, l'utilisation cosmétique selon l'invention est pour réduire les odeurs corporelles au niveau des régions axillaires, des zones péri-anales, des pieds et/ou des aréoles mammaires.

De préférence, le chitosan natif et/ou l'acide salicylique ou l'un de ses dérivés et/ou l'alpha hydroxy acide présente(nt) une ou des caractéristiques telles que définies dans la description ci-dessous.

De préférence, la composition de l'utilisation cosmétique est une composition selon l'invention.

La présente invention concerne également un procédé cosmétique de prévention et/ou de réduction des odeurs corporelles, comprenant la mise en contact d'une matière kératinique, de préférence la peau, avec une composition comprenant, dans un milieu aqueux physiologiquement acceptable :
(a) du chitosan natif,
(b) de l'acide salicylique ou l'un de ses dérivés, et
(c) au moins un alpha hydroxy acide.

De préférence, la composition selon l'invention est appliquée sur la peau au niveau des régions axillaires, des zones péri-anales, des pieds et/ou des aréoles mammaires.

De préférence, le chitosan natif et/ou l'acide salicylique ou l'un de ses dérivés et/ou l'alpha hydroxy acide présente(nt) une ou des caractéristiques telles que définies dans la description ci-dessous.

De préférence, la composition du procédé cosmétique est une composition selon l'invention.

De préférence, la composition selon l'invention est substantiellement exempte de polymère (méth)acrylique. De préférence, la composition selon l'invention est substantiellement exempte de silicone.

Par « substantiellement exempte de polymère (méth)acrylique », on entend que la composition comprend moins de 1 % en poids par rapport au poids total de la composition, de préférence moins de 0,5% en poids, de préférence moins de 0,3% en poids, préférentiellement 0,1% en poids de polymère (méth)acrylique. De préférence, la composition est totalement exempte de polymère (méth)acrylique. Par polymère (méth)acrylique, on entend un polymère comprenant au moins un monomère d'acide acrylique ou au moins un monomère d'acide méthacrylique.

Par « substantiellement exempte de silicone », on entend que la composition comprend moins de 1 % en poids par rapport au poids total de la composition, de préférence moins de 0,5% en poids, de préférence moins de 0,3% en poids, préférentiellement 0,1% en poids de silicone. De préférence, la composition est totalement exempte de silicone. Par silicone, on entend tout composé siliconé.

### Chitosan

La composition selon l'invention comprend au moins un chitosan natif.

De préférence, le chitosan natif a un poids moléculaire strictement supérieur à 3000 Daltons (3 kDa), de préférence supérieur ou égal à 10 kDa, de préférence supérieur ou égal à 15 kDa, de préférence supérieur ou égal à 20 kDa. De préférence, le chitosan natif a un poids moléculaire compris entre 10 kDa et 2 MDa, de préférence compris entre 15 kDa et 1,5 MDa, de préférence compris entre 20 kDa et 300 kDa, de préférence compris entre 20 kDa et 200 kDa.

Le chitosan (ou chitosane) est très peu répandu dans la nature. Il n'est signalé que dans les exosquelettes de certains insectes comme les reines des termites et dans les parois cellulaires d'une classe particulière de champignons, les zygomycètes.

Le chitosan est obtenu par désacétylation de la chitine. La chitine est un polysaccharide composé de plusieurs unités N-acétyl-D-glucosamine reliées entre elles par une liaison de type β (1,4).

La structure chimique idéale du chitosane est un enchaînement de monomères β-D-glucosamine reliés par une liaison glycosidique (1→4).

Par « chitosan » selon l'invention, on entend tout copolymère formé d'unités constitutives N-acétyl-D-glucosamine et D-glucosamine, dont le degré d'acétylation est inférieur à 90%. Le chitosan est constitué des unités sucres glucosamine (unités désacétylées) et d'unités N-acétyl-D-glucosamine (unités acétylées) reliées entre elles par des liaisons de type β (1,4) et constitue un polymère du type Poly(N-acetyl-D-glucosamine)-poly(D-glucosamine).

De préférence, le degré d'acétylation du chitosan est inférieur ou égal à 80%, de préférence inférieur ou égal à 70%, de préférence inférieur ou égal à 60%, de préférence inférieur ou égal à 50%, de préférence inférieur ou égal à 35%, de préférence inférieur ou égal à 25%, de préférence inférieur ou égal à 15%, de préférence compris entre 1% et 50%.

Le degré d'acétylation est le pourcentage d'unités acétylées par rapport au nombre d'unités totales, il peut être déterminé par spectroscopie infrarouge à transformée de Fourier (IR-TF) ou par un titrage par une base forte.

Le chitosan de l'invention est de préférence un polysaccharide préparé à partir d'une origine fongique. Il est notamment extrait et purifié à partir de sources fongiques alimentaires ou biotechnologique sûres et abondantes tels que *Agaricus bisporus* ou *Aspergillus niger.*

Le chitosan de l'invention est de préférence issu du mycélium d'un champignon du type Ascomycète, et en particulier *d'Aspergillus niger* et/ou d'un champignon Basidiomycète, et en particulier *Lentinula edodes* (shiitake) et/ou *Agaricus bisporus.* De préférence le champignon est *Aspergillus niger.*

Le chitosan peut être d'origine OGM, mais de préférence est d'origine non OGM.

Le chitosan selon l'invention est natif, c'est-à-dire qu'il n'est pas modifié. Il ne contient notamment pas de modification chimique.

Une méthode de préparation du chitosan est celle décrite dans la demande WO03068824.

De préférence, le chitosan utilisé dans l'invention est sous forme de poudre. Il est notamment commercialisé par Kitozyme sous le nom Kiosmetine ou Kionutrime.

Le chitosan est de préférence présent en une quantité allant de 0,1% à 10% en poids, de préférence de 0,2% à 5% en poids, de préférence de 0,3% à 3% en poids, de préférence de 0,5% à 2% en poids, de préférence de 0,8% à 1,6% en poids par rapport au poids total de la composition.

### Acide salicylique ou l'un de ses dérivés

La composition selon l'invention comprend de l'acide salicylique ou l'un de ses dérivés.

Les dérivés d'acide salicyliques selon l'invention sont de préférence des dérivés lipophiles. Par dérivé lipophile de l'acide salicylique, on entend tout dérivé de l'acide salicylique non soluble à 1 % en poids dans l'eau et à 25°C.

Les dérivés d'acide salicylique sont de préférence de formule (I) suivante : 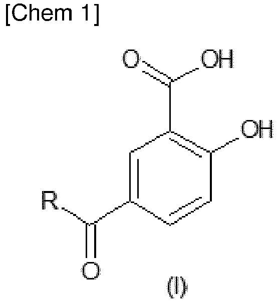 dans laquelle R désigne une chaîne aliphatique saturée, linéaire, ramifiée ou cyclique, ayant de 2 à 22 atomes de carbone; une chaîne insaturée, linéaire ou ramifiée, ayant de 2 à 22 atomes de carbone contenant une ou plusieurs doubles liaisons pouvant être conjuguées ; un noyau aromatique lié au radical carbonyle directement ou par l'intermédiaire de chaînes aliphatiques saturées ou insaturées ayant de 2 à 8 atomes de carbone; lesdits groupes pouvant être substitués par un ou plusieurs substituants, identiques ou différents, choisis parmi (A) les atomes d'halogène (B) le groupe trifluorométhyle, (C) des groupes hydroxyle sous forme libre ou estérifiée par un acide ayant de 1 à 6 atomes de carbone ou (D) une fonction carboxyle sous forme libre ou estérifiée par un alcool inférieur ayant de 1 à 6 atomes de carbone, ainsi que leurs sels obtenus par salification par une base minérale ou organique.

De préférence, R désigne une chaîne aliphatique saturée, linéaire, ramifiée ou cyclique contenant de 3 à 11 atomes de carbone ; une chaîne insaturée, linéaire ou ramifiée, contenant de 3 à 17 atomes de carbone et comprenant une ou plusieurs doubles liaisons conjuguées ou non ; lesdites chaînes hydrocarbonées pouvant être substituées par un ou plusieurs substituants, identiques ou différents, choisis parmi (A) les atomes d'halogène (B) le groupe trifluorométhyle, (C) des groupes hydroxyle sous forme libre ou estérifiée par un acide ayant de 1 à 6 atomes de carbone ou (D) une fonction carboxyle sous forme libre ou estérifiée par un alcool inférieur ayant de 1 à 6 atomes de carbone ; ainsi que leurs sels obtenus par salification par une base minérale ou organique.

Préférentiellement, R est un groupe alkyle en C3-C11, linéaire ou ramifié.

Parmi les composés de formule (I) particulièrement préférés, on peut citer l'acide n-octanoyl-5-salicylique (ou acide capryloyl salicylique) ; l'acide n-décanoyl-5-salicylique ; l'acide n-dodécanoyl-5-salicylique ; l'acide n-heptyloxy-5-salicylique et leurs sels correspondants.

Préférentiellement, la composition selon l'invention comprend de l'acide salicylique ou de l'acide n-octanoyl-5-salicylique, avantageusement comprend de l'acide N-octanoyl-5-salicylique.

Les sels des composés de la formule (I) peuvent être obtenus par salification avec une base minérale ou organique. A titre d'exemple de base minérale, on peut citer les hydroxydes de métaux alcalins ou alcalino-terreux tels que l'hydroxyde de sodium, l'hydroxyde de potassium ou l'ammoniaque.

Parmi les bases organiques, on peut citer les amines et les alcanolamines. Les sels quaternaires tels que ceux décrits dans le brevet FR 2.607.498 sont particulièrement intéressants.

De préférence, la composition selon l'invention comprend, par rapport au poids total de la composition, de 0,01% à 5% en poids, de préférence de 0,02% à 3% en poids, de préférence de 0,05% à 2% en poids, de préférence de 0,1 % à 1% en poids, de préférence de 0,15% à 0,5% en poids, d'acide salicylique ou l'un de ses dérivés.

De préférence, le ratio pondéral entre la teneur en poids en chitosan et la teneur en poids en acide salicylique ou l'un de ses dérivés va de 1,1 à 10, de préférence de 2 à 8, avantageusement de 4 à 6.

### Alpha hydroxy acide (AHA)

La composition selon la présente invention comprend au moins un alpha hydroxy acide (AHA).

Par alpha hydroxy acide, on entend selon la présente invention un acide carboxylique ayant au moins une fonction hydroxy occupant une position alpha sur ledit acide (carbone adjacent à une fonction acide carboxylique). Cet acide peut se présenter dans la composition finale sous forme d'acide libre et/ou sous la forme de l'un de ses sels associés (sels avec une base organique ou un alcalin notamment), en particulier selon le pH final imposé à la composition.

Les alpha hydroxy acides sont par exemple choisis parmi l'acide lactique, l'acide citrique, l'acide méthyllactique, l'acide glucuronique, l'acide glycolique, l'acide pyruvique, l'acide 2-hydroxy-butanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxy-nonanoïque, l'acide 2-hydroxydécanoïque, l'acide 2-hydroxyundécanoïque, l'acide 2-hydroxydodécanoïque, l'acide 2-hydroxytétradécanoïque, l'acide 2-hydroxy-hexadécanoïque, l'acide 2-hydroxyoctadécanoïque, l'acide 2-hydroxytétra-cosanoïque, l'acide 2-hydroxyeïcosanoïque ; l'acide mandélique ; l'acide phényllactique ; l'acide gluconique ; l'acide galacturonique ; l'acide aleuritique ; l'acide ribonique ; l'acide tartronique ; l'acide tartrique ; l'acide malique ; l'acide fumarique ; leurs sels et leurs mélanges.

Selon un mode préféré, l'alpha hydroxyacide est choisi parmi l'acide lactique, l'acide gluconique, l'acide citrique, l'acide malique, l'acide glycolique, l'acide tartrique et leurs sels. Plus particulièrement, l'alpha hydroxyacide est choisi parmi l'acide lactique, l'acide citrique, leurs sels et leurs mélanges.

Avantageusement, l'alpha hydroxyacide est l'acide lactique, ses sels et leurs mélanges.

Le ou les alpha hydroxyacides peuvent être présents en une quantité allant par exemple de 0,05% à 5% en poids, de préférence de 0,1% à 3% en poids, de préférence de 0,2% à 1% en poids, de préférence de 0,3% à 0,8% en poids par rapport au poids total de la composition.

De préférence, le ratio pondéral entre la teneur en poids en chitosan et la teneur en poids, de préférence la teneur totale en poids, en alpha hydroxyacide(s) va de 1,1 à 5, de préférence de 1,2 à 4, avantageusement de 1,5 à 2,5.

Un tel ratio permet avantageusement d'améliorer la solubilisation du chitosan, et donc d'obtenir l'effet booster de l'activité antimicrobienne de l'acide salicylique et ses dérivés.

De préférence, le ratio pondéral entre la teneur en poids, de préférence la teneur totale en poids, en alpha hydroxyacide(s) et la teneur en poids, de préférence la teneur totale en poids, en acide salicylique ou l'un de ses dérivés va de 1,1 à 5, de préférence de 1,2 à 4, avantageusement de 1,5 à 3.

### Polyols

La composition selon la présente invention peut également comprendre au moins un polyol.

Le terme « polyol » désigne ici un alcool ayant deux groupes hydroxy ou plus et n'englobe pas de saccharide ou de dérivé de celui-ci. Le dérivé d'un saccharide inclut un alcool de sucre obtenu en réduisant un ou plusieurs groupes carbonyles d'un saccharide, ainsi qu'un saccharide ou un alcool de sucre dans lequel le ou les atomes d'hydrogène dans un ou plusieurs groupes hydroxy de celui-ci a (ont) été remplacé(s) par au moins un substituant tel qu'un groupe alkyle, un groupe hydroxyalkyle, un groupe alcoxy, un groupe acyle ou un groupe carbonyle.

Le polyol peut être un polyol en C₂₋₂₄, de préférence un polyol en C₂₋₉, comprenant au moins 2 groupes hydroxy, et de préférence 2 à 5 groupes hydroxy.

Le polyol peut être un polyol naturel ou synthétique. Le polyol peut avoir une structure moléculaire linéaire, ramifiée ou cyclique.

Le polyol peut être choisi parmi les glycérines et leurs dérivés, ainsi que les glycols et leurs dérivés. Le polyol peut être choisi dans le groupe consistant en glycérine, diglycérine, polyglycérine, éthylèneglycol, diéthylèneglycol, propylèneglycol, dipropylèneglycol, butylèneglycol, pentylèneglycol, hexylèneglycol, 1,3-propanediol, 1,4-butanediol, 1,5-pentanediol, et les polyéthylèneglycol, notamment ayant de 5 à 50 groupes d'oxyde d'éthylène et leurs mélanges ; plus particulièrement choisi parmi la glycérine, le propylèneglycol, le dipropylèneglycol, le butylèneglycol et leurs mélanges.

Le polyol est de préférence du propylène glycol.

Le polyol peut être présent dans la composition selon l'invention en une teneur allant de 1% à 50% en poids, par rapport au poids total de la composition, de préférence allant de 5% à 40% en poids, de préférence allant de 8% à 30% en poids, de préférence allant de 10% à 20% en poids.

### pH de la composition

De préférence, la composition selon l'invention présente un pH inférieur ou égal à 7, de préférence inférieur ou égal à 6,5, de préférence inférieur ou égal à 6,3. Avantageusement, le pH de la composition est compris entre 3 et 6,3, de préférence compris entre 4 et 6,3.

### Milieu aqueux physiologiquement acceptable

Le milieu aqueux physiologiquement acceptable comprend de l'eau.

L'eau utilisée peut être de l'eau déminéralisée stérile et/ou une eau florale telle que de l'eau de rose, de l'eau de bleuet, de l'eau de camomille ou de l'eau de tilleul, et/ou une eau thermale ou minérale naturelle, comme par exemple : l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avène

La première composition comprend de préférence au moins 50% en poids d'eau par rapport au poids total de la première composition, de préférence au moins 60% en poids.

La composition selon l'invention comprend de préférence de 50% à 95% en poids d'eau par rapport au poids total de la composition, plus préférentiellement de 60% à 90% en poids.

Par ailleurs, la composition de l'invention peut aussi comprendre une phase organique non-miscible dans l'eau appelée phase grasse. Celle-ci comprend en général un ou plusieurs composés hydrophobes qui rendent ladite phase non-miscible dans l'eau. Ladite phase peut être liquide (en l'absence d'agent structurant) à température ambiante (20-25°C). De manière préférentielle, la phase organique liquide organique non-miscible dans l'eau conforme à l'invention comprend généralement au moins une huile, volatile ou non volatile, et éventuellement au moins un agent structurant. Par « huile », on entend un corps gras liquide à température ambiante (25 °C) et pression atmosphérique (760mm de Hg soit 10⁵ Pa). L'huile peut être volatile ou non volatile.

La composition de l'invention peut également comprendre au moins un corps gras solide. De préférence, ce corps gras solide est choisi parmi les cires et les corps gras pâteux et leurs mélanges.

La composition selon l'invention peut également comprendre au moins un actif déodorant additionnel, différent du chitosan natif, de l'acide salicylique ou l'un de ses dérivés et de l'alpha hydroxy acide de la composition selon l'invention.

Par « actif déodorant », on entend dans le cadre de la présente invention tout actif qui, à lui seul, a pour effet de masquer, absorber, améliorer et/ou diminuer l'odeur désagréable résultant de la décomposition de la sueur humaine.

A titre illustratif de ces actifs déodorants additionnels peuvent notamment être cités le zinc néodécanoate, le sel de zinc de l'acide L-Pyrrolidone Carboxylique (zinc PCA), le zinc gluconate ou le chlorhydrate d'octénidine.

Les actifs déodorants additionnels peuvent être présents de préférence dans les compositions selon l'invention dans des concentrations pondérales allant 0,01 à 10% en poids par rapport au poids total de la composition.

La composition selon l'invention peut en outre comprendre des adjuvants cosmétiques choisis parmi les opacifiants, les stabilisants, les conservateurs, les parfums, les actifs cosmétiques, les charges, les agents de suspension, les séquestrants, les matières colorantes ou tout autre ingrédient habituellement utilisé en cosmétique pour ce type d'application. Bien entendu, l'homme de métier veillera à choisir ce ou ces éventuels composés complémentaires de manière telle que les propriétés avantageuses de la composition selon l'invention, ne soient pas, ou substantiellement pas, altérées par la ou les adjonctions envisagées.

### Formes galéniques

La composition selon l'invention peut indépendamment se présenter sous toutes les formes galéniques classiquement utilisées pour une application topique et notamment sous forme de gels aqueux, de solutions aqueuses ou hydroalcooliques. Elles peuvent, par ajout d'une phase grasse ou huileuse, se présenter sous forme de dispersions du type lotion, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou des dispersions cire/phase aqueuse. La composition de l'invention peut être notamment conditionnées sous forme pressurisée dans un dispositif aérosol ou dans un flacon pompe ; conditionnées dans un dispositif muni d'une paroi ajourée notamment une grille ; conditionnées dans un dispositif muni d'un applicateur à billes ("roll-on ») ; conditionnées sous forme de bâtonnets (sticks), sous forme de poudre libre ou compactée. Elles contiennent à cet égard les ingrédients généralement utilisés dans ce type de produits et bien connus de l'homme de l'art.

La composition conforme à l'invention peut être obtenue de manière classique par l'homme du métier.

Les expressions « compris entre ... et ... » et « allant de ... à ... » doivent se comprendre bornes incluses, sauf si le contraire est spécifié.

Des exemples concrets, mais nullement limitatifs, illustrant l'invention, vont maintenant être donnés.

### EXEMPLES

### Exemple 1 : Préparation d'une composition selon l'invention et de compositions comparatives

Les compositions C1 et C2 selon l'invention et les compositions comparatives C3* et C5* du tableau 1 ci-dessous sont préparées par le protocole suivant :
- Préparation de la phase A en dispersant le chitosan dans l'eau puis en ajoutant l'acide lactique (pH = 5),
- Préparation de la phase B en solubilisant l'acide capryloyl salicylique dans le propylène glycol,
- Introduction de la phase B dans la phase A sous agitation jusqu'à obtention d'un liquide marron mousse en surface, de pH = 4,6.

Pour la composition C4*, on prépare la phase A en préparant le gel de xanthane par mélange dans l'eau des deux gommes. On prépare la phase B en solubilisant l'acide capryloyl salicylique avec le propylène glycol. Puis on introduit la phase B dans la phase A et on ajoute le caprylyl glycol.

**[Table 1]**

| Ingrédient (% p/p) | C1 | C2 | C3* | C4* | C5* |
|---|---|---|---|---|---|
| Eau | Qsp 100 | Qsp 100 | Qsp 100 | Qsp 100 | Qsp 100 |
| Propylène glycol | 15 | 15 | 15 | 15 | - |
| **Chitosan (27 kDa)** | **1** | - | **1** | - | **0,1** |
| **Chitosan (110 kDa)** | - | 1 | - | - | - |
| **Acide capryloyl salicylique** | **0,2** | **0,2** | - | **0,2** | - |
| **Acide lactique** | **0,45** | **0,45** | **0,45** | - | **0,07** |
| Gomme xanthane déshydratée | - | - | - | 0,29 | - |
| Gomme xanthane | - | - | - | 0,39 | - |
| Caprylyl glycol | - | - | - | 0,5 | - |

### Exemple 2 : Evaluation de l'activité antimicrobienne des compositions C1, C2 et C3* à C5*

L'activité antimicrobienne des compositions C1 et C2 selon l'invention, et des compositions comparatives C3* à C5* a été évaluées en utilisant la bactérie *Corynebacterium xerosis,* selon le protocole suivant :
Dans un milieu de culture adapté, l'effet biocide des formules est évalué sur les souches d'intérêt après 2h, 6h et 24h de contact en comparaison avec un placebo (i.e. milieu sans formule). A chaque temps de contact, la réduction logarithmique du nombre de viables est mesurée.

La souche modèle « d'odeur axillaire » est *Corynebacterium freneyi* CIP 52.16 (ex *Corynebacterium xerosis).*

Cette souche est mise en contact avec la formule à tester dans un milieu de culture dans les proportions suivantes :
- 10% de l'inoculum à 10⁸ germes/ml,
- 10% de la formule à tester, et
- 80% de milieu de culture (bouillon Trypcase Soja).

En parallèle, un témoin de croissance, dans lequel la formule est remplacée par un diluant, est préparé dans les mêmes conditions.

Les échantillons sont placés dans un incubateur à une température de 35°C (température de croissance optimale pour la souche) avec une agitation continue pendant le test.

Après 2, 6 et 24 heures de temps de contact, le nombre de microorganismes viables restant dans le mélange est évalué.

Les résultats sont exprimés en logarithme du nombre de micro-organismes par millilitre de mélange.

Le résultat obtenu avec la formule à tester est comparé aux résultats du témoin de croissance et la différence est exprimée en nombre de Log différence à T 24 heures.

Les résultats obtenus sont présentés dans le tableau 2 suivant, indiquant le nombre de bactéries *Corynebacterium xerosis* revivifiables en fonction du temps, et de la composition utilisée (le seuil de détection étant de 2,3).

**[Table 2]**

| Composition | Temps (heures) | | | |
|---|---|---|---|---|
| | 0 | 2 | 6 | 24 |
| Contrôle | 7,6 | 7,6 | 7,7 | 8,4 |
| C1 | **7,4** | **4,3** | **3,7** | **1,6** |
| C2 | **7,4** | **4,1** | **4,0** | **1,7** |
| C3* | 7,4 | 4,1 | 3,9 | 3,0 |
| C4* | 7,4 | 6,9 | 6,7 | 5,9 |
| C5* | 7,1 | 4,1 | 2,8 | 5,6 |

L'activité antimicrobienne de la composition comparative C4* comprenant uniquement de l'acide capryloyl salicylique et la composition comparative C3* comprenant uniquement du chitosan diminue dans le temps, en particulier entre 6 et 24 heures. Au contraire, la composition C1 selon l'invention comprenant l'association de chitosan et l'acide capryloyl salicylique démontre une excellente activité antimicrobienne, même après 24 heures. En effet, le nombre de bactéries revivifiables étant en dessous du seuil de détection, cela signifie qu'au bout de 24h, l'ensemble des bactéries sont détruites par la composition C1 selon l'invention.

Ces résultats démontrent donc que l'ajout de chitosan dans une composition comprenant de l'acide capryloyl salicylique permet de booster son activité antimicrobienne.

## Revendications

1. Composition, comprenant, dans un milieu aqueux physiologiquement acceptable :
(a) du chitosan natif,
(b) de l'acide salicylique ou l'un de ses dérivés, et
(c) au moins un alpha hydroxy acide,
dans laquelle la teneur en poids en chitosan est strictement supérieure à la teneur en poids en alpha hydroxy acide, et la teneur en poids en alpha hydroxy acide est strictement supérieure à la teneur en poids en acide salicylique ou l'un de ses dérivés, les teneurs en poids étant définies par rapport au poids total de la composition.

2. Composition selon la revendication 1, comprenant, par rapport au poids total de composition, de 0,1% à 10% en poids, de préférence de 0,2% à 5% en poids, de préférence de 0,3% à 3% en poids, de préférence de 0,5% à 2% en poids, de préférence de 0,8% à 1,6% en poids de chitosan natif.

3. Composition selon la revendication 1 ou 2, dans laquelle le chitosan natif (a) a un poids moléculaire strictement supérieur à 3000 Daltons, de préférence supérieur ou égal à 10 kDa, de préférence supérieur ou égal à 15 kDa, de préférence supérieur ou égal à 20 kDa, de préférence compris entre 10 kDa et 2 MDa, de préférence compris entre 15 kDa et 1,5 MDa, de préférence compris entre 20 kDa et 300 kDa; de préférence compris entre 20 kDa et 200 kDa et/ou le degré d'acétylation du chitosan est inférieur ou égal à 50%, de préférence inférieur ou égal à 35%, de préférence inférieur ou égal à 25%, de préférence inférieur ou égal à 15%.

4. Composition selon l'une quelconque des revendications précédentes, dans laquelle le chitosan (a) est extrait et purifié à partir de sources fongiques alimentaires ou biotechnologique telles que *Agaricus bisporus* ou *Aspergillus niger,* de préférence à partir *d'Aspergillus niger.*

5. Composition selon l'une quelconque des revendications précédentes, comprenant, par rapport au poids total de composition, de 0,01% à 5% en poids, de préférence de 0,02% à 3% en poids, de préférence de 0,05% à 2% en poids, de préférence de 0,1% à 1% en poids, de préférence de 0,15% à 0,5% en poids d'acide salicylique ou l'un de ses dérivés.

6. Composition selon l'une quelconque des revendications précédentes, comprenant un dérivé d'acide salicylique de formule (I) : 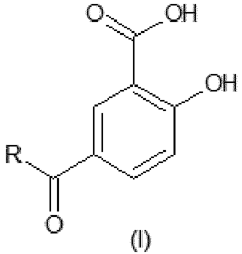
dans laquelle R désigne une chaîne aliphatique saturée, linéaire, ramifiée ou cyclique, ayant de 2 à 22 atomes de carbone; une chaîne insaturée, linéaire ou ramifiée, ayant de 2 à 22 atomes de carbone contenant une ou plusieurs doubles liaisons pouvant être conjuguées ; un noyau aromatique lié au radical carbonyle directement ou par l'intermédiaire de chaînes aliphatiques saturées ou insaturées ayant de 2 à 8 atomes de carbone; lesdits groupes pouvant être substitués par un ou plusieurs substituants, identiques ou différents, choisis parmi (A) les atomes d'halogène (B) le groupe trifluorométhyle, (C) des groupes hydroxyle sous forme libre ou estérifiée par un acide ayant de 1 à 6 atomes de carbone ou (D) une fonction carboxyle sous forme libre ou estérifiée par un alcool inférieur ayant de 1 à 6 atomes de carbone,
ainsi que leurs sels obtenus par salification par une base minérale ou organique,
de préférence le dérivé d'acide salicylique de formule (I) est choisi parmi l'acide n-octanoyl-5-salicylique (ou acide capryloyl salicylique) ; l'acide n-décanoyl-5-salicylique ; l'acide n-dodécanoyl-5-salicylique ; l'acide n-heptyloxy-5-salicylique et leurs sels correspondants ; de préférence la composition comprend l'acide salicylique ou l'acide n-octanoyl-5-salicylique, avantageusement comprend l'acide N-octanoyl-5-salicylique.

7. Composition selon l'une quelconque des revendications précédentes, dans laquelle l'alpha hydroxy acide est choisi parmi l'acide lactique, l'acide gluconique, l'acide citrique, l'acide malique, l'acide glycolique, l'acide tartrique et leurs sels.

8. Composition selon l'une quelconque des revendications précédentes, comprenant, par rapport au poids total de composition, de 0,05% à 5% en poids, de préférence de 0,1% à 3% en poids, de préférence de 0,2% à 1% en poids, de préférence de 0,3% à 0,8% en poids d'alpha hydroxy acide.

9. Composition selon l'une quelconque des revendications précédentes, qui présente un pH inférieur ou égal à 7, de préférence inférieur ou égal à 6,5, de préférence inférieur ou égal à 6,3, avantageusement le pH de la composition est compris entre 3 et 6,3, de préférence compris entre 4 et 6,3.

10. Composition selon l'une quelconque des revendications précédentes, comprenant en outre au moins un polyol en une quantité allant de 1 à 50 % en poids, par rapport au poids total de la composition.

11. Composition selon l'une des revendications précédentes qui est substantiellement exempte de polymère (méth)acrylique et/ou de silicone ; de préférence la composition comprend moins de 1% en poids par rapport au poids total de la composition, de préférence moins de 0,5% en poids, de préférence moins de 0,3% en poids, préférentiellement 0,1% en poids de polymère (méth)acrylique, de préférence la composition est totalement exempte de polymère (méth)acrylique ; de préférence la composition comprend moins de 1% en poids par rapport au poids total de la composition, de préférence moins de 0,5% en poids, de préférence moins de 0,3% en poids, préférentiellement 0,1% en poids de silicone, de préférence la composition est totalement exempte de silicone.

12. Utilisation cosmétique d'une composition comprenant, dans un milieu aqueux physiologiquement acceptable :
(a) du chitosan natif,
(b) de l'acide salicylique ou l'un de ses dérivés, et
(c) au moins un alpha hydroxy acide,
pour prévenir et/ou réduire les odeurs corporelles.

13. Procédé cosmétique de prévention et/ou de réduction des odeurs corporelles, comprenant la mise en contact d'une matière kératinique, de préférence la peau, avec une composition comprenant,
(a) du chitosan natif,
(b) de l'acide salicylique ou l'un de ses dérivés, et
(c) au moins un alpha hydroxy acide.
